(19) Europäisches Patentamt / European Patent Office / Office européen des brevets

(11) **EP 4 575 227 A1**

(12) **DEMANDE DE BREVET EUROPEEN**

(43) Date de publication:
   **25.06.2025  Bulletin 2025/26**

(21) Numéro de dépôt: **24222684.3**

(22) Date de dépôt: **21.12.2024**

(51) Classification Internationale des Brevets (IPC):
   *F04B 19/00* (2006.01)   *F04F 7/00* (2006.01)
   *A61M 5/142* (2006.01)   *A61M 37/00* (2006.01)
   *A61N 7/00* (2006.01)   *B06B 1/06* (2006.01)
   *F04B 43/04* (2006.01)   *A61M 5/00* (2006.01)

(52) Classification Coopérative des Brevets (CPC):
   **F04B 19/006; A61M 5/00; A61M 5/142;
   A61M 37/00; B06B 1/06; F04B 43/043; F04F 7/00**

(84) Etats contractants désignés:
   **AL AT BE BG CH CY CZ DE DK EE ES FI FR GB
   GR HR HU IE IS IT LI LT LU LV MC ME MK MT NL
   NO PL PT RO RS SE SI SK SM TR**
   Etats d'extension désignés:
   **BA**
   Etats de validation désignés:
   **GE KH MA MD TN**

(30) Priorité: **21.12.2023  FR 2314942**

(71) Demandeur: **Commissariat à l'Energie Atomique
   et aux Energies
   Alternatives
   75015 Paris (FR)**

(72) Inventeurs:
   • **NIKOLOVSKI, Jean-Pierre
     38054 Grenoble cedex 09 (FR)**
   • **DELEVOYE, Elisabeth
     38054 Grenoble cedex 09 (FR)**

(74) Mandataire: **INNOV-GROUP
   209 Avenue Berthelot
   69007 Lyon (FR)**

(54) **POMPE PÉRISTALTIQUE ACTIONNÉE PAR DES TRANSDUCTEURS PIÉZOÉLECTRIQUES**

(57)    Pompe péristaltique, destinée à pomper un liquide le long d'un capillaire (2), le pompage résultant d'une contrainte exercée sur le capillaire, successivement le long une direction de pompage, la pompe comportant :
- K transducteurs piézoélectriques annulaires ($11_k$), s'étendant autour d'un axe central ($\Delta$),
- K résonateurs ($10_k$), formés d'un matériau solide déformable, chaque résonateur étant relié à un transducteur piézoélectrique, et s'étendant autour de l'axe central, en s'amincissant vers l'axe central, chaque résonateur étant configuré pour se déformer sous l'effet d'une polarisation du transducteur piézoélectrique auquel il est relié ;
- une unité de commande (20), configurée pour polariser chaque transducteur piézoélectrique
la pompe étant caractérisée en ce que :
- chaque résonateur est relié à un manchon ($3_k$), s'étendant autour de l'axe central ;
- chaque résonateur est agencé de façon que les manchons de chaque résonateur sont alignés le long d'un axe central.

**Fig.1A**

EP 4 575 227 A1

**Description**

## DOMAINE TECHNIQUE

Le domaine technique de l'invention est une pompe péristaltique configurée pour être actionnée par un transducteur piézoélectrique.

## ART ANTERIEUR

La plupart des pompes mettent en oeuvre des pièces mobiles, ce qui peut générer un problème de fiabilité, d'usure, et de limitation de la compacité. Dans le domaine de la santé, des pompes péristaltiques sont d'usage courant. Cependant, l'écrasement répété d'un tuyau, qui entraîne le déplacement du liquide, peut entraîner une usure prématurée du tuyau.

La demande de brevet WO2013/41700 décrit une pompe, pouvant être implantable, non péristaltique, à actionnement piézoélectrique. Un manchon, disposé au centre d'un résonateur subit une flexion, sous l'effet d'une déformation tournante du résonateur, engendrée par des transducteurs piézoélectriques activés selon une fréquence ultrasonique. La flexion du manchon génère un effet de pompage, qui entraîne l'expulsion du fluide. Une réduction de la section transversale du résonateur, au voisinage du manchon ou le long de ce dernier, permet d'amplifier les vibrations se propageant jusqu'au manchon. Une telle pompe est efficace. Cependant, il a été constaté que le manchon subit une flexion répétée, ce qui peut entraîner une usure. D'autre part, la pompe est destinée à être couplée à un circuit fluidique. La maîtrise du débit dépend de l'amplitude de vibration du manchon qui doit être asservie à la charge mécanique du circuit fluidique, ce qui n'est pas aisé.

En particulier, on cherche à ce que le principe de pompage, la fréquence et l'amplitude de la vibration ultrasonore de pompage soient moins dépendants du couplage mécanique de la pompe avec le circuit fluidique.

Un autre objectif est de concevoir une pompe péristaltique, permettant d'effectuer un pompage, selon un débit maîtrisé, avec une dépense énergétique optimisée, et pouvant être particulièrement compacte.

## EXPOSE DE L'INVENTION

Un objet de l'invention est une pompe péristaltique, destinée à pomper un liquide le long d'un capillaire, d'une contrainte exercée sur le capillaire, successivement le long une direction de pompage, la pompe comportant :

- K transducteurs piézoélectriques annulaires, s'étendant autour d'un axe central, chaque transducteur piézoélectrique et configuré pour être polarisé par une électrode de polarisation, K étant un entier supérieur ou égal à 2

- K résonateurs, formés d'un matériau solide déformable, chaque résonateur étant relié à un transducteur piézoélectrique, et s'étendant autour de l'axe central, en s'amincissant vers l'axe central, chaque résonateur étant configuré pour se déformer sous l'effet d'une polarisation du transducteur piézoélectrique auquel il est relié ;
- une unité de commande, configurée pour polariser chaque électrode de polarisation selon une tension de polarisation, modulée selon une fréquence de modulation;

la pompe étant caractérisée en ce que :

- chaque résonateur est relié à un manchon, s'étendant autour de l'axe central ;
- chaque résonateur est agencé de façon que les manchons de chaque résonateur sont alignés le long d'un axe central ;
- l'unité de commande est configurée pour polariser successivement chaque résonateur, en fonction de leurs rangs respectifs, de façon que sous l'effet de la polarisation, une déformation successive de chaque résonateur se produit, la déformation se propageant jusqu'au manchon, et entraînant une déformation successive du capillaire (2) disposé entre les manchons, autour de l'axe central.

La contrainte peut notamment être une compression et/ou un déplacement selon l'axe central. La fréquence de modulation peut notamment être supérieure à 20 kHz.

Selon une possibilité :

- chaque résonateur comporte une portion périphérique, délimitée par deux faces planes, et une portion centrale, s'étendant entre la portion périphérique et le manchon, la portion centrale s'amincissant vers le manchon ;
- chaque transducteur piézoélectrique s'étend le long d'une face plane.

Selon une possibilité, la face plane le long de laquelle s'étend chaque transducteur périphérique est délimitée par un épaulement, le transducteur piézoélectrique étant inséré en force contre l'épaulement, de façon que l'épaulement contourne le transducteur piézoélectrique.

Selon une possibilité, chaque résonateur est couplé à au moins un transducteur piézoélectrique annulaire, le transducteur piézoélectrique annulaire s'étendant autour du résonateur, de façon à engendrer une compression radiale du résonateur, en direction de l'axe central.

Chaque transducteur piézoélectrique peut être inséré en force autour du résonateur auquel il est relié.

Selon une possibilité, chaque résonateur est couplé à deux transducteurs piézoélectriques annulaires, s'étendant autour du résonateur, et décalés selon une direction parallèle à l'axe central, l'unité centrale étant configurée pour alimenter les transducteurs piézoélectriques de façon à engendrer un déplacement du manchon, le long de l'axe central et/ou une compression du manchon perpendiculairement à l'axe central.

Selon une possibilité, chaque transducteur piézoélectrique présentant un moment dipolaire :

- les moments dipolaires des transducteurs piézoélectriques couplés au même résonateur sont orientés selon une même direction, auquel cas l'unité de commande est configurée pour alimenter lesdits transducteurs piézoélectriques en phase;
- les moments dipolaires des transducteurs piézoélectriques couplés au même résonateur sont orientés selon deux directions opposés, auquel cas l'unité de commande est configurée pour alimenter lesdits transducteurs piézoélectriques en opposition de phase ;
- de façon à engendrer une compression radiale du manchon prédominant sur un déplacement du manchon le long de l'axe central.

Selon une possibilité, chaque transducteur piézoélectrique présentant un moment dipolaire :

- les moments dipolaires des transducteurs piézoélectriques couplés au même résonateur sont orientés selon une même direction, auquel cas l'unité de commande est configurée pour alimenter lesdits transducteurs piézoélectriques en opposition de phase ;
- les moments dipolaires des transducteurs piézoélectriques couplés au même résonateur sont orientés selon deux directions opposés, auquel cas l'unité de commande est configurée pour alimenter lesdits transducteurs piézoélectriques en phase ;
- de façon à engendrer un déplacement du manchon, le long de l'axe central, prédominant sur une compression du manchon.

Selon une possibilité, l'unité de commande est configurée pour alimenter lesdits transducteurs piézoélectriques selon un décalage de phase prédéterminé, compris entre 0° et 180°, de façon à obtenir un ratio prédéterminé entre le déplacement du manchon selon l'axe central sur la compression du manchon.

La pompe peut comporter le capillaire, le capillaire étant par exemple inséré en force entre chaque manchon. Le capillaire peut être rigide

L'invention sera mieux comprise à la lecture de l'exposé des exemples de réalisation présentés, dans la suite de la description, en lien avec les figures listées ci-dessous.

## FIGURES

Les figures 1A et 1B montrent un mode de réalisation d'une pompe péristaltique.
La figure 2 représente une variante de la configuration décrite en lien avec les figures 1A et 1B
La figure 3A montre un autre mode de réalisation d'une pompe péristaltique.
La figure 3B montre un exemple de transducteur piézoélectrique annulaire.
La figure 4 montre un autre mode de réalisation d'une pompe péristaltique.

## EXPOSE DE MODES DE REALISATION PARTICULIERS

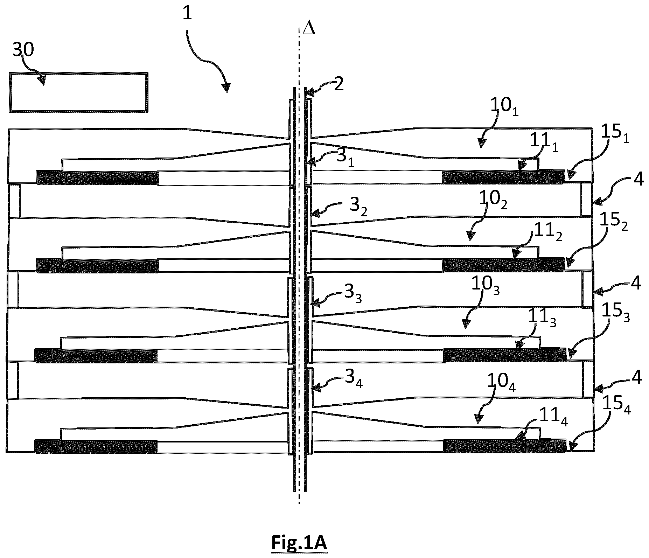

La figure 1A décrit une pompe péristaltique, dans laquelle un effet de pompage est obtenu en appliquant une compression progressive le long d'un capillaire 2. Le capillaire 2 est coaxial d'un axe central

La pompe comporte K résonateurs $10_k$, k étant un rang assigné à chaque résonateur. Le nombre minimal de résonateurs est de 2, et le nombre optimal de résonateurs est compris entre 3 et SDans l'exemple représenté, K = 4. On dispose ainsi de 4 résonateurs identiques $10_1$, $10_2$, $10_3$, $10_4$, alignés le long de l'axe central. Chaque résonateur s'étend selon une forme annulaire, entre une partie périphérique, comportant des portions plates, d'épaisseur constante, et une partie s'amincissant vers l'axe central. Chaque résonateur s'amincit, de préférence progressivement, jusqu'à un manchon $3_k$, cylindrique, et s'étendant autour de l'axe central. L'amincissement progressif permet d'augmenter les efforts de déformation transférés au manchon.

Le capillaire 2 est disposé le long de l'axe central, entre chaque manchon. De préférence, le capillaire 2 est rigide afin de pouvoir être introduit en force dans les manchons. Une insertion en force minimise les pertes aux interfaces sous forme de réflexion parasite de l'énergie mécanique ou une dissipation par frottement. De plus, une insertion en force ne nécessite pas de moyen de serrage. Le capillaire peut être formé du même matériau que le résonateur.

Chaque résonateur comporte un épaulement $15_k$, permettant d'enserrer le transducteur auquel il est couplé, de façon à exercer une précontrainte. Ainsi, chaque épaulement contourne le transducteur piézoélectrique qu'il enserre. Chaque transducteur piézoélectrique peut ainsi être maintenu sans colle, par la simple contrainte mécanique. Cela permet d'éviter une dégradation, dans le temps, d'un collage, sous l'effet du vieillissement.

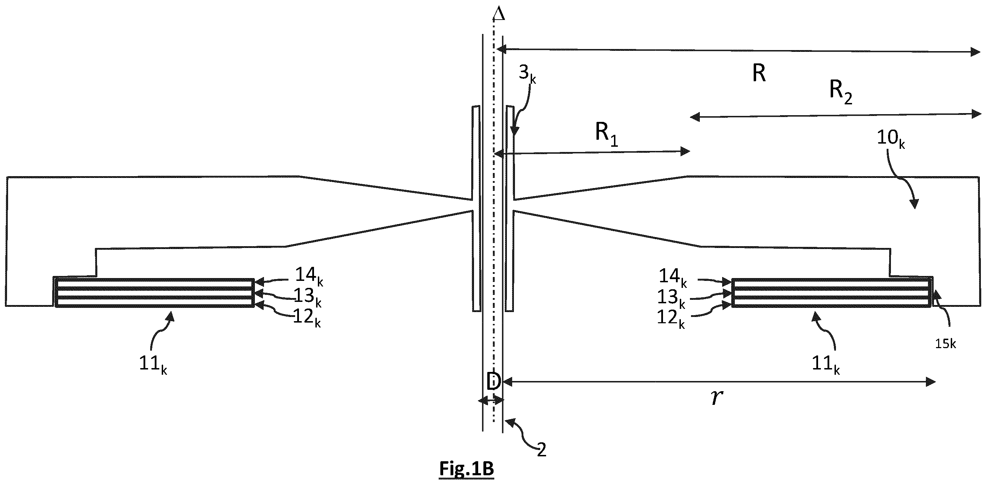

Chaque résonateur $10_k$ est couplé à un transducteur piézoélectrique annulaire $11_k$. La figure 1B représente un détail du couplage mécanique d'un transducteur piézoélectrique $11_k$ annulaire, relié à un résona-

teur $10_k$. Le transducteur piézoélectrique $11_k$ comporte une couche d'un matériau piézoélectrique $13_k$ s'étendant entre au moins une électrode $12_k$ et une contre électrode $14_k$ d'autre part. Lorsque le résonateur $10_k$ est réalisé dans un matériau conducteur électrique, l'électrode $12_k$ a de préférence un diamètre externe légèrement inférieur, par exemple de 0.2mm à 0.5 mm, à la contre électrode $14_k$ de façon à éviter un court-circuit électrique entre les électrodes $12_k$ et $14_k$ via l'épaulement $15_k$. Le résonateur $10_k$ est symétrique par rapport à l'axe central $\Delta$. Le résonateur $10_k$ est annulaire, autour de l'axe central $\Delta$. Il s'amincit vers ce dernier. Ainsi, son épaisseur, définie parallèlement à l'axe central $\Delta$, diminue en fonction de la distance à l'axe central $\Delta$. L'amincissement permet d'augmenter l'amplitude des vibrations se propageant dans le résonateur, vers le manchon.

Dans cette configuration les transducteurs piézoélectriques ont une faible épaisseur, typiquement comprise entre 0.05 mm et 5 mm, préférentiellement 0.5 mm pour un rayon r de 25 mm et 0.2 mm pour un rayon r inférieur à 10 mm, ce qui maximise le champ électrique, ce dernier pouvant être de l'ordre de 300 V/mm. Cela permet d'augmenter la contrainte mécanique, celle-ci étant directement proportionnelle au champ électrique.

Chaque couche de matériau piézoélectrique $13_k$ peut être formée d'un matériau de type PZT (Zirconate titanate de plomb), en particulier les références PZ26, PZ27, PZ46 et PZ29 de Ferroperm. De préférence, les coefficient d33 et d31, qui rendent compte du coefficient de la déformation observée pour un champ électrique appliqué (également perçu comme une densité de charges récoltées pour une contrainte appliquée), sont respectivement :

- d'au moins 200 pC/N et de préférence au-delà de 570 pC/N pour le coefficient d33 quantifiant la réponse du matériau piézoélectrique dans une direction parallèle à la direction du champ électrique appliqué.
- et d'au moins de 50 pC/N et préférentiellement de l'ordre de -240 pC/N. pour d31, qui quantifie la réponse du matériau piézoélectrique dans une direction perpendiculaire à la direction du champ électrique appliqué.

Le rayon externe R de chaque résonateur $10_k$, défini autour de l'axe central $\Delta$, peut s'étendre jusqu'à 50 mm, ou davantage. La portion plane de chaque résonateur s'étend au-delà d'un premier rayon $R_1$, inférieur au rayon externe R précédemment défini. En deçà du premier rayon $R_1$, chaque résonateur présente une portion s'amincissant en direction de l'axe central $\Delta$. Le premier rayon $R_1$ est par exemple égal à 50% du rayon externe R du résonateur.

Chaque résonateur peut s'étendre selon une épaisseur de préférence inférieure à 5 mm, par exemple 1 ou 2 mm. L'épaisseur est définie parallèlement à l'axe longitudinal.

Le résonateur $10_k$ s'étend jusqu'à un premier manchon $3_k$, cylindrique, coaxial de l'axe central $\Delta$. Le diamètre D du manchon $3_k$ est par exemple compris entre environ 0.2 mm et 2 mm et préférentiellement proche de 1 mm. Le manchon $3_k$ est de préférence formé par un prolongement du résonateur $10_k$, le résonateur et le manchon formant une pièce monolithique. Le rayon du manchon forme un rayon interne du premier résonateur $10_k$. La hauteur de chaque manchon $3_k$ est de préférence inférieure à une longueur d'onde des ondes se propageant dans le manchon. La hauteur de chaque manchon $3_k$ est de préférence égale à une demi-longueur d'onde. La longueur d'onde dépend de l'épaisseur du manchon et de la fréquence de résonance. En pratique la hauteur du manchon est inférieure à 1 mm et de préférence inférieure ou égale l'épaisseur du résonateur dans la portion appliquée contre le transducteur piézoélectrique.

Ceci permet que le manchon de chaque résonateur soit aussi proche que possible que le manchon d'un autre résonateur, sans être en contact direct. L'écartement entre deux manchons adjacents peut être d'au moins 0.05 mm.

Chaque résonateur est formé d'un matériau solide déformable, pouvant être de type métal (titane, inox, aluminium ou alliage d'aluminium, laiton, ou autre alliage à base de cuivre, ou alliage à base de nickel), matériau inorganique (verre), matériau organique (PEEK), alumine.

La pompe comporte une unité de commande 20, reliée à chaque première électrode $12_k$ de façon à leur appliquer une tension de polarisation modulée en fréquence. La fréquence de modulation dépend de la dimension et du matériau formant chaque résonateur. La fréquence est la même pour chaque transducteur piézoélectrique. Lorsque le rayon de chaque résonateur est de 25 mm, la fréquence de modulation peut être d'environ 25 kHz. Lorsque le rayon de chaque résonateur est de 12.5 mm, la fréquence de modulation peut être d'environ 50 kHz. Lorsque le rayon de chaque résonateur est de 6.5 mm, la fréquence de modulation peut être d'environ 100 kHz. Dans tous les cas, la fréquence de modulation est de préférence ultrasonique, de façon à éviter une génération d'un son audible.

Chaque contre-électrode $14_k$ est reliée à un potentiel fixe, qui peut être commun à toutes les contre-électrodes (par exemple une masse), ou à indépendant du potentiel auquel est reliée une autre électrode, chaque potentiel indépendant étant par exemple une masse flottante.

Chaque résonateur $10_k$ est configuré pour être déformé par une onde de compression agissant sur chaque manchon, de façon à comprimer radialement le capillaire. Sous l'effet de la compression, le diamètre interne de chaque manchon oscille selon une fréquence de résonance radiale de compression. Selon une possibilité, chaque manchon peut se déplacer parallèlement à l'axe du capillaire selon une fréquence de résonance radiale de flexion.

Plus précisément, les dimensions du résonateur étant finies, un élément de surface situé sur la paroi interne délimitant chaque manchon oscille en décrivant une ellipse à chaque période de résonance. Le grand axe $s_z$ de l'ellipse est parallèle à l'axe central $\Delta$ selon une fréquence de résonance radiale de flexion. Il y a donc un léger effet de roulement imprimé au capillaire. Le résonateur $10_k$ peut vibrer à sa fréquence fondamentale ou à une fréquence harmonique. Dans ce dernier cas, le rapport entre le grand axe $s_z$ et le petit axe s, de l'ellipse parcourue devient plus proche de 1. Le mouvement devient plus circulaire pour une fréquence harmonique plus élevée.

Les résonateurs peuvent être séparés les uns des autres par des entretoises 4. Cela permet de rigidifier l'empilement.

L'épaisseur de l'empilement peut être comprise entre 5 mm et 10 mm.

Les transducteurs sont déphasés selon l'axe longitudinal $\Delta$, de façon que la compression se déplace le long de l'axe central. Le déphasage temporel est de $\frac{2\pi}{K}T$, où T correspond à la période au cours de laquelle chaque transducteur a été actionné. On estime que la fréquence de résonance est au maximum de 150 kHz pour une fréquence fondamentale.

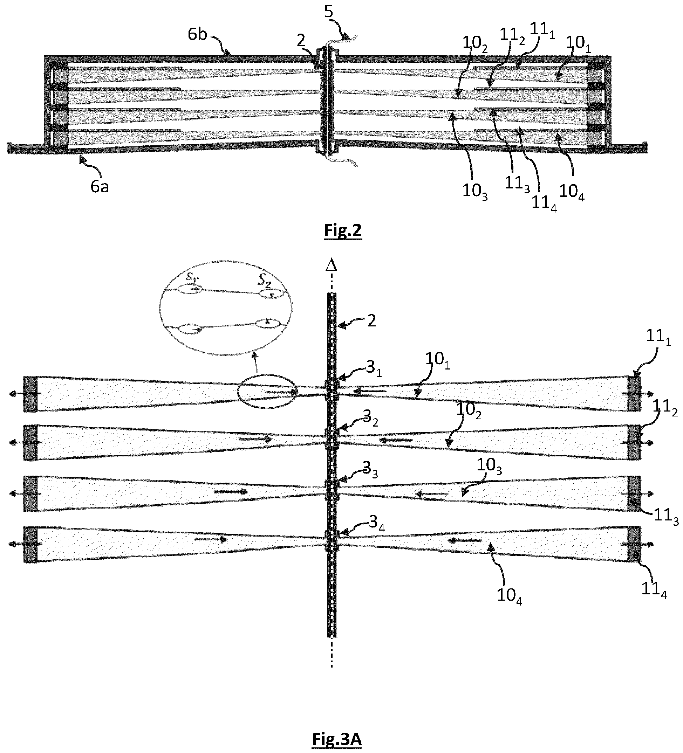

La figure 2 illustre une variante du mode de réalisation décrit en lien avec les figures 1A et 1B, dans laquelle l'empilement est logé dans un boîtier. Chaque extrémité du capillaire est conique, ce qui facilite un raccordement avec un capillaire souple 5, par exemple en silicone. Dans cet exemple, l'assemblage formé par les résonateurs est maintenu sur un socle $6_a$ sur lequel est clipsé un couvercle $6_b$.

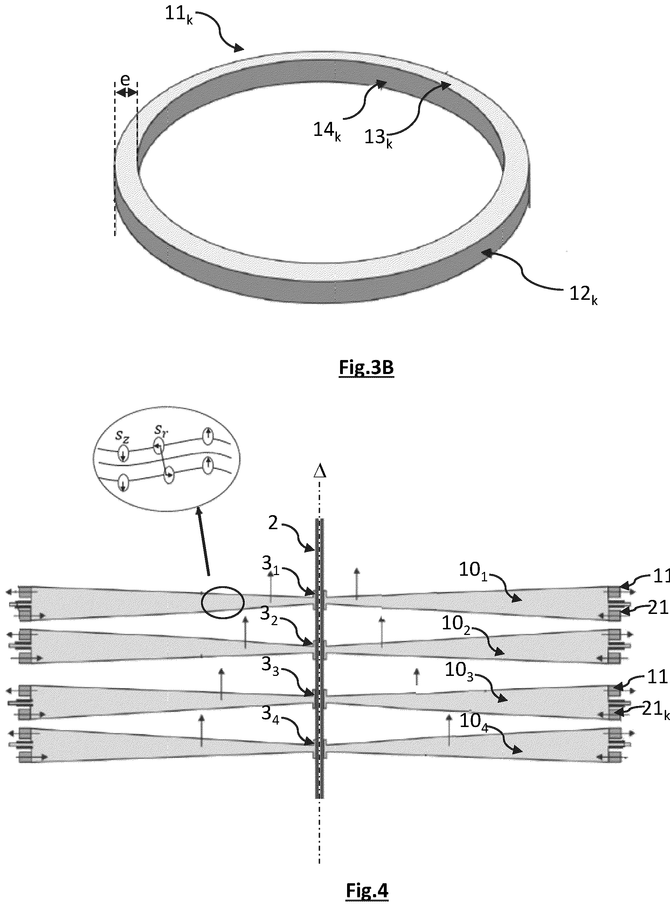

Les figures 3A et 3B illustrent un mode de réalisation proche du mode de réalisation des figures 1A et 1B, dans lequel chaque transducteur piézoélectrique est un anneau disposé autour du résonateur auquel il est couplé. Une telle configuration est propice à la génération d'ondes de Lamb symétriques se propageant radialement, en convergeant vers l'axe central. Cela permet de concentrer les efforts de compression au niveau du manchon. Dans ce cas, l'ellipse parcourue par le déplacement d'un élément de surface du manchon à l'interface avec le capillaire a son grand axe orienté perpendiculairement à l'axe central $\Delta$ du capillaire. Les transducteurs sont déphasés (ou activés selon un décalage temporel) selon l'axe central $\Delta$, de façon que la compression associée au grand axe de l'ellipse se déplace le long de l'axe central. Le déphasage ou décalage temporel est de $\frac{2\pi}{K}T$, où T correspond à la période au cours de laquelle chaque transducteur a été actionné.

Le recours à des transducteurs piézoélectriques disposés à la périphérie de résonateurs permet d'augmenter la fréquence, ces derniers pouvant résonner en résonance d'épaisseur à une fréquence élevée, en pratique au-delà du mégahertz ou une fréquence plus basse si l'on exploite une résonance de compression radiale du disque aminci. Cela permet d'utiliser un capillaire plus fin proche du dixième à quelques dixièmes de millimètres. Une telle configuration est adaptée à une délivrance de faibles doses de principe actif, ou à une pompe implantée ou à une dispense de faibles volumes de fluides tels que des catalyseurs ou des produits onéreux ou à des apports lents et bien contrôlés de poudres ou de produits divers (gaz, lubrifiant, etc..) nécessaires à une production particulière ou à une maintenance.

Sur la figure 3B, on a représenté un transducteur piézoélectrique annulaire : l'électrode 12k et la contre électrode 14k sont annulaires, de même que la couche piézoélectrique 13k. Dans cet exemple, l'épaisseur de la couche piézoélectrique $13_k$ est de 1 mm, le diamètre de l'électrode $12_k$ étant par exemple égal à 22 mm. Afin d'assurer une isolation électrique, entre l'électrode $12_k$ et le résonateur $10_k$ auquel elle est rattachée, la couche piézoélectrique $13_k$ peut être biseautée (biseau non représenté) sur une profondeur d'au moins 0.1 mm sur les bords de l'électrode externe 12k. Dans la direction radiale, la largeur de la couche piézoélectrique peut être comprise entre 0.5 mm et 1 mm. La fréquence de résonance pour une épaisseur de 1 mm est d'environ 2MHz.

La figure 4 montre une configuration proche de celle décrite en lien avec les figures 3A et 3B. Dans cette configuration, chaque résonateur est couplé à deux transducteurs piézoélectriques annulaires $11_1$, $21_1$, activés en opposition de phase, et disposés autour du résonateur. Une telle configuration est propice à la génération d'ondes de Lamb antisymétriques se propageant radialement, convergeant dans la région amincie entraînant un cisaillement du capillaire 2, le cisaillement se propageant selon une direction prédéterminée. Dans ce cas, l'ellipse parcourue par un élément de surface à l'interface entre le manchon et le capillaire présente un grand axe $s_z$ parallèle à l'axe central $\Delta$ du capillaire.

De même que dans le mode de réalisation précédent, les transducteurs couplés à deux résonateurs adjacents sont déphasés selon l'axe longitudinal $\Delta$, de façon à faire propager la contrainte de cisaillement de proche en proche le long du capillaire. Le déphasage (ou décalage) temporel est de $\frac{2\pi}{K}T$, où T correspond à la période au cours de laquelle chaque transducteur a été actionné. Au cours d'une même phase, le premier transducteur $11_k$ d'un résonateur $10_k$ est activé, de façon à induire un flambage dans un sens du résonateur dans sa région centrale, tandis que le deuxième transducteur $21_k$ d'un résonateur $10_k$ est activé, de façon à induire un flambage dans le même sens du résonateur et doit donc être déphasé de $\pi$, au niveau de l'axe central. Ceci est obtenu si les électrodes externes respectives des transducteurs $11_k$ et $21_k$ sont soumises à la même tension d'excitation électrique et si les moments dipolaires électriques des matériaux piézoélectriques respectifs des transducteurs $11_k$ et $21_k$ sont de sens opposés comme indiqué par les flèches traversant les transducteurs qui sont reportées sur la figure 4. On obtient ainsi un pompage par effet péristaltique de cisaillement, du fait de la progressivité du cisaillement du capillaire 2, qui engendre un déplacement du fluide par viscosité et incompressibilité du fluide le long de l'axe central. L'efficacité du pompage est renforcée par une composante de déplacement s, dans le plan de chaque résonateur, correspondant au petit axe de l'ellipse de déplacement et induisant une compression du tube capillaire. Les modes acoustiques symétrique et antisymétrique initiés en périphérie des résonateurs des figures 3A et 4 comprennent tous les deux des composantes de déplacement de compression s, et de cisaillement $s_z$ du capillaire correspondant aux grand axe et petit axe de l'ellipse parcourue par un vecteur déplacement, mais avec une dominante de compression en s, pour le mode symétrique et de cisaillement en $s_z$ pour le mode antisymétrique. Ces deux modes de vibration sont considérés comme péristaltiques.

La configuration représentée sur la figure 4 permet également de former des modes acoustiques symétriques, tels que schématisés sur la figure 3A, lorsque les électrodes externes $12_k$ des deux transducteurs piézoélectriques $11_k$, $21_k$ reliés à chaque résonateur $10_k$ sont en opposition de phase et si les moments dipolaires électriques des transducteurs piézoélectriques $11_k$ et $21_k$ sont de sens contraires comme indiqués sur la figure 4.

Lorsque les moments dipolaires des transducteurs $11_k$ et $21_k$ sont orientés dans le même sens, et que les tensions d'excitation sont en opposition de phase, on obtient un mode de vibration antisymétrique tel que précédemment décrit. Lorsque les tensions d'excitation sont en phase, on obtient un mode de vibration symétrique.

Un mode de vibration symétrique a pour effet de comprimer le capillaire et produire un déplacement de l'interface capillaire/manchon parcourant une ellipse avec un grand axe orthogonal à l'axe Δ. Cette vibration se transmet dans la paroi du capillaire sous la forme d'une onde de flexion à symétrie axiale par rapport à l'axe Δ.

Un mode de vibration antisymétrique a pour effet de produire un déplacement de l'interface capillaire/manchon dont le grand axe de l'ellipse est parallèle à l'axe Δ ce qui se traduit par un cisaillement du capillaire en respectant la symétrie axiale par rapport à l'axe Δ.

Il en résulte que si l'on fait varier le déphasage électrique entre les deux transducteurs $11_k$ et $21_k$ de 0 à 180° on passe continûment d'une génération prépondérante d'un mode de déformation symétrique à une génération prépondérante d'un mode antisymétrique par rapport au plan médian du résonateur $10_k$. Les deux modes de vibration symétrique et antisymétrique sont créés simultanément mais dans des proportions différentes selon le déphasage et avec des vitesses de phase distinctes et plus grande pour le mode symétrique que pour le mode antisymétrique. Sachant que ces modes de vibration peuvent coexister simultanément et de façon élastique dans le résonateur, il suffit d'imposer un déphasage d'excitation électrique particulier compris entre 0 et 180°C pour obtenir une vibration de la paroi du capillaire de façon que chaque élément de surface du capillaire se déplace selon une ellipse, dont le grand axe est susceptible de varier d'une orientation perpendiculaire à l'axe Δ à une orientation parallèle en l'axe Δ en fonction du déphasage que l'on aura choisi entre les deux transducteurs $11_k$ et $21_k$.

Quelles que soient les configurations, la tension de polarisation crête / crête peut varier par exemple de quelques volts à plusieurs centaines de volts, la tension jouant sur l'amplitude de la composante de déformation hors plan du résonateur. L'amplitude de cette composante de déformation joue sur le volume de fluide à l'interface interne du capillaire subissant l'effet de roulement elliptique qui engendre son entrainement et donc sur la pression de pompage.

**Revendications**

1. Pompe péristaltique, destinée à pomper un liquide le long d'un capillaire (2), le pompage résultant d'une contrainte exercée sur le capillaire, successivement le long une direction de pompage, la pompe comportant :

   - K transducteurs piézoélectriques annulaires ($11_k$), s'étendant autour d'un axe central (Δ), chaque transducteur piézoélectrique et configuré pour être polarisé par une électrode de polarisation ($12_k$), K étant un entier supérieur ou égal à 2

   - K résonateurs ($10_k$), formés d'un matériau solide déformable, chaque résonateur étant relié à un transducteur piézoélectrique, et s'étendant autour de l'axe central, en s'amincissant vers l'axe central, chaque résonateur étant configuré pour se déformer sous l'effet d'une polarisation du transducteur piézoélectrique auquel il est relié ;

   - une unité de commande (20), configurée pour polariser chaque électrode de polarisation selon une tension de polarisation, modulée selon une fréquence de modulation;

   la pompe étant **caractérisée en ce que** :

   - chaque résonateur est relié à un manchon ($3_k$), s'étendant autour de l'axe central ;
   - chaque résonateur est agencé de façon que les manchons de chaque résonateur sont alignés le long d'un axe central ;
   - l'unité de commande (20) est configurée pour polariser successivement chaque résonateur, en fonction de leurs rangs respectifs, de façon que sous l'effet de la polarisation, une déformation successive de chaque résonateur se produit, la déformation se propageant jusqu'au manchon, et entraînant une déformation successive du capillaire (2) disposé entre les manchons, autour de l'axe central.

2. Pompe selon la revendication 1, dans laquelle la contrainte est une compression et/ou un déplacement selon l'axe central.

3. Pompe selon l'une quelconque des revendications précédentes, dans laquelle la fréquence de modulation est supérieure à 20 kHz.

4. Pompe selon l'une quelconque des revendications précédentes, dans laquelle :

   - chaque résonateur comporte une portion périphérique, délimitée par deux faces planes, et une portion centrale, s'étendant entre la portion périphérique et le manchon, la portion centrale s'amincissant vers le manchon ;
   - chaque transducteur piézoélectrique s'étend le long d'une desdites faces planes.

5. Pompe selon l'une quelconque des revendications précédentes, dans laquelle la face plane le long de laquelle s'étend chaque transducteur périphérique est délimitée par un épaulement ($15_k$), le transducteur piézoélectrique étant inséré en force contre l'épaulement, de façon que l'épaulement contourne le transducteur piézoélectrique.

6. Pompe selon l'une quelconque des revendications précédentes, dans laquelle chaque résonateur est couplé à au moins un transducteur piézoélectrique annulaire, le transducteur piézoélectrique annulaire s'étendant autour du résonateur, de façon à engendrer une compression radiale du résonateur, en direction de l'axe central.

7. Pompe selon la revendication 6, dans laquelle chaque transducteur piézoélectrique est inséré en force autour du résonateur auquel il est relié.

8. Pompe selon l'une quelconque des revendications précédentes dans laquelle chaque résonateur est couplé à deux transducteurs piézoélectriques annulaires, s'étendant autour du résonateur, et décalés selon une direction parallèle à l'axe central, l'unité centrale étant configurée pour alimenter les transducteurs piézoélectriques de façon à engendrer un déplacement du manchon, le long de l'axe central et/ou une compression du manchon perpendiculairement à l'axe central.

9. Pompe selon la revendication 8, dans laquelle chaque transducteur piézoélectrique présentant un moment dipolaire :

   - les moments dipolaires des transducteurs piézoélectriques couplés au même résonateur sont orientés selon une même direction, auquel cas l'unité de commande est configurée pour alimenter lesdits transducteurs piézoélectriques en phase;
   - les moments dipolaires des transducteurs piézoélectriques couplés au même résonateur sont orientés selon deux directions opposés, auquel cas l'unité de commande est configurée pour alimenter lesdits transducteurs piézoélectriques en opposition de phase ;
   - de façon à engendrer une compression radiale du manchon prédominant sur un déplacement du manchon le long de l'axe central.

10. Pompe selon la revendication 8 dans laquelle chaque transducteur piézoélectrique présentant un moment dipolaire :

   - les moments dipolaires des transducteurs piézoélectriques couplés au même résonateur sont orientés selon une même direction, auquel cas l'unité de commande est configurée pour alimenter lesdits transducteurs piézoélectriques en opposition de phase ;
   - les moments dipolaires des transducteurs piézoélectriques couplés au même résonateur sont orientés selon deux directions opposés, auquel cas l'unité de commande est configurée pour alimenter lesdits transducteurs piézoélectriques en phase ;
   - de façon à engendrer un déplacement du manchon, le long de l'axe central, prédominant sur une compression du manchon.

11. Pompe selon la revendication 8, dans lequel l'unité de commande est configurée pour alimenter lesdits transducteurs piézoélectriques selon un décalage de phase prédéterminé, compris entre 0° et 180°, de façon à obtenir un ratio prédéterminé entre le déplacement du manchon selon l'axe central sur la compression du manchon.

12. Pompe selon l'une des revendications précédentes, comportant le capillaire (2), le capillaire étant inséré en force entre chaque manchon.

13. Pompe selon la revendication 12, dans lequel le capillaire est rigide.

**Fig.1A**

**Fig.1B**

**Fig.2**

**Fig.3A**

**Fig.3B**

**Fig.4**

Europäisches Patentamt

European Patent Office

Office européen des brevets

# RAPPORT DE RECHERCHE EUROPEENNE

Numéro de la demande

EP 24 22 2684

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A,D | WO 2013/041700 A1 (COMMISSARIAT ENERGIE ATOMIQUE [FR]) 28 mars 2013 (2013-03-28) * figures 1-5 * * page 18, ligne 19 - page 24, ligne 17 * * revendications 1-7 * ----- | 1-13 | INV. F04B19/00 F04F7/00 A61M5/142 A61M37/00 A61N7/00 |
| A | WO 2011/109735 A2 (CORNELL UNIVERSITY; LEWIS, G.K.; OLBRICHT, W.L. [US]) 9 septembre 2011 (2011-09-09) * figure 1A * * figures 15B, 15C, 15D * * alinéa [0087] * * alinéa [0134] - alinéa [0136] * * alinéa [0160] * ----- | 1-13 | B06B1/06 F04B43/04 A61M5/00 |
| A | WO 03/057275 A2 (EKOS CORPORATION [US]) 17 juillet 2003 (2003-07-17) * figures 3-7C * * page 8, ligne 8 - page 11, ligne 23 * * page 20, ligne 10 - ligne 30 * ----- | 1-13 | |

DOMAINES TECHNIQUES RECHERCHES (IPC)

F04B
F04F
A61M
A61N
B06B

Le présent rapport a été établi pour toutes les revendications

| Lieu de la recherche | Date d'achèvement de la recherche | Examinateur |
|---|---|---|
| Munich | 9 janvier 2025 | Gnüchtel, Frank |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 24 22 2684

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-01-2025

| Document brevet cité au rapport de recherche | Date de publication | Membre(s) de la famille de brevet(s) | | Date de publication |
|---|---|---|---|---|
| WO 2013041700 A1 | 28-03-2013 | EP | 2758664 A1 | 30-07-2014 |
| | | FR | 2980535 A1 | 29-03-2013 |
| | | US | 2014377091 A1 | 25-12-2014 |
| | | WO | 2013041700 A1 | 28-03-2013 |
| WO 2011109735 A2 | 09-09-2011 | US | 2013046230 A1 | 21-02-2013 |
| | | WO | 2011109735 A2 | 09-09-2011 |
| WO 03057275 A2 | 17-07-2003 | AU | 2002367296 A1 | 24-07-2003 |
| | | US | 2004073114 A1 | 15-04-2004 |
| | | WO | 03057275 A2 | 17-07-2003 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 201341700 A